# EUROPEAN PATENT APPLICATION

(11) **EP 1 316 791 A2**
(43) Date of publication of application: **04.06.2003**
(21) Application number: 02258229.0
(22) Date of filing: 29.11.2002
(51) Int. Cl.: G01N 3/56

(54) **Reciprocating abrasion tester and method for evaluating abrasion resistance**

(30) Priority: 29.11.2001 JP 2001364654
(71) Applicant: Nippon Sheet Glass Co., Ltd., Osaka-shi, Osaka 541-8559 (JP)
(72) Inventor: Kamitani, Kazutaka, c/o Nippon Sheet Glass Co. Ltd, Osaka-shi, Osaka 541-8559 (JP); Muromoto, Kenzo, c/o Nippon Sheet Glass Co., Ltd., Osaka-shi, Osaka 541-8559 (JP); Takeuchi, Yoshitaka, c/o Nippon Sheet Glass Co Ltd, Osaka-shi, Osaka 541-8559 (JP)
(74) Representative: Power, Philippa Louise

(57) **Abstract**

The present invention provides a reciprocating abrasion tester (1) capable of conducting an abrasion test that exhibits small variations in evaluation even when conducted with respect to an automobile windshield glass using a wiper blade so as to conform to the actual abrasion conditions. This tester includes: a motor (12); reciprocating abrasion devices (2) connected to the motor, and a loading member (33) for applying a load (34) to the sliding member (32). Each of the reciprocating abrasion devices includes a crank (21), a reciprocating arm (22), and a sliding member for causing abrasion of a sample (4) surface. Reciprocation cycles of the reciprocating abrasion devices differ from each other in phase.

## Description

The present invention relates generally to a reciprocating abrasion tester, particularly to a reciprocating abrasion tester employing a wiper blade as a sliding member.

In general, Taber-type rotary abrasion testers frequently are used for the measurement of abrasion resistance of materials and films. Also, Suga-type abrasion testers and reciprocating abrasion testers available from Shinto Scientific Co., Ltd. widely are used for the measurement. See JP 11(1999)-130470 A filed by the applicant of the present invention and JP 2000-143296 A, for example.

With the above-mentioned various reciprocating abrasion testers, an abrasion test is conducted with a sliding member being moved at a sliding speed of about 80 to 160 mm/sec. These reciprocating abrasion testers are designed so as to permit the replacement of the sliding member.

In the case where an abrasion test is conducted with respect to an automobile windshield glass with a water repellent film formed thereon, it is preferable that the sliding member slides under the conditions similar to those during actual use. On this account, it is required that the sliding member slides on the surface of the automobile windshield glass more than several tens of thousands times at a considerably high sliding speed, e.g., at least 600 mm/sec. The term "sliding speed" as used herein refers to a distance travelled by the sliding member per unit time and thus is an average value.

Therefore, in the above-mentioned reciprocating abrasion testers, an abrasion test takes a long time if the sliding member slides on the surface of the windshield glass the required number of times. In addition, it cannot be said that the above-mentioned reciprocating abrasion testers reproduce an actual abrasion mode since they fail to provide a sufficiently high sliding speed.

Further, in an abrasion test conducted with respect to an automobile windshield glass, evaluating the abrasion at the portions where a wiper blade turns around (hereinafter, referred to as the "turn-around portions") is particularly important. Therefore, it is preferable to conduct an abrasion test using a wiper blade as a sliding member so as to conform to the conditions during actual use.

The present invention provides a reciprocating abrasion tester capable of conducting a reliable abrasion test in evaluation even when conducted with respect to an automobile windshield glass using a wiper blade so as to conform to the actual abrasion conditions.

### (Preliminary Consideration)

The Inventors first considered modifying a commercially available reciprocating abrasion tester. Specifically, the Inventors considered replacing the sliding member used therein with a wiper blade, and further, replacing the motor used therein with a motor capable of rotating at a higher speed to increase a sliding speed of the sliding member.

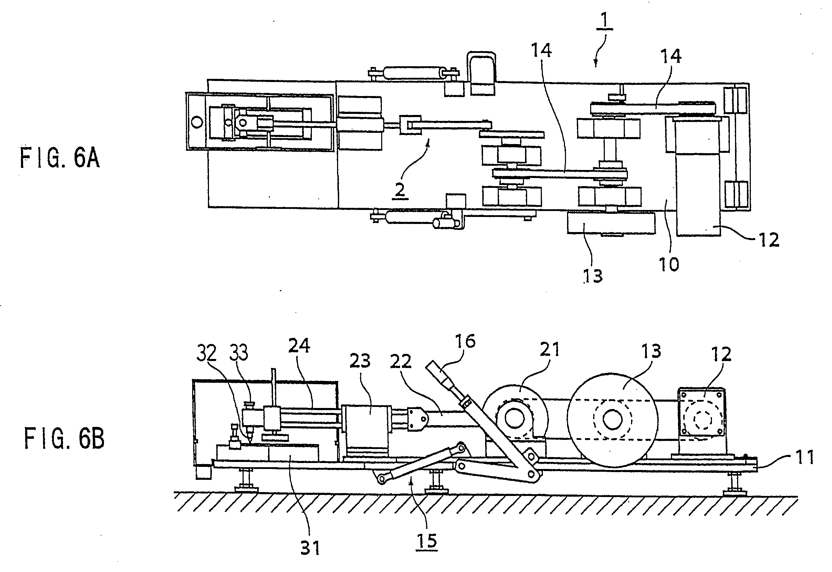

FIGs. 6A and 6B show one example of the thus-modified abrasion tester. In the abrasion tester shown in FIGs. 6A and 6B, a wiper blade reciprocates on the surface of a glass while being rotated at a constant speed by a motor and with a certain load being applied thereto. By using such an abrasion tester, the resistance of a glass against abrasion caused by a wiper can be evaluated while reproducing the same load and the same sliding speed as those of an actual wiper of an automobile.

The abrasion tester 1 includes a single abrasion reciprocating device 2. The other numerals will be explained below.

However, an abrasion test conducted using this abrasion tester exhibits considerable variations in evaluation. Even in the case where samples formed of the same material are evaluated under the same conditions, they may differ considerably in degree of abrasion. It is considered that the high sliding speed of the sliding member (i.e., wiper blade) causes such variations.

The reason for this is as follows. When the wiper blade slides at a high sliding speed, a considerable difference in friction force applied to the wiper blade is caused between the turn-around portions and other portions. Also, in the abrasion tester, a friction force, especially the one generated between a shaft moving horizontally and a bearing supporting the shaft, changes greatly when the wiper blade turns around. Accordingly, strong vibrations are generated in the abrasion tester itself when the wiper blade turns around.

Consequently, in addition to the abrasion typical of the turn-around portions, abrasion caused by the bounding action of the wiper blade resulting from vibrations occurs in the vicinity of the turn-around portions. In addition, the bounding action of the wiper blade varies from one test to another due to subtle misalignment of a movable shaft with respect to a bearing supporting the shaft and to the abrasion of the movable shaft and the bearing. These are considered to be the reason why the results of the abrasion test vary.

Besides, due to the great changes in the friction force as described above, the rotational speed of the motor becomes unstable so that smooth sliding action of the wiper blade is prone to be interrupted.

Especially, in the case where the wiper blade is used as the sliding member, the direction in which the blade is deflected is reversed when the wiper blade turns around. Further, the inventors of the present invention confirmed that vibrations are too strong when the wiper blade slides at the sliding speed of 200 mm/sec or more.

Moreover, the abrasion tester shown in FIGs. 6A and 6B can test only one sample in one abrasion test because of its structure.

From a first aspect therefore, the present invention provides a reciprocating abrasion tester comprising:
a motor;
reciprocating abrasion devices connected to the motor, each of the reciprocating abrasion devices comprising a sliding member for causing abrasion of a sample surface; and
a loading member for applying a load to the sliding member,
wherein reciprocation cycles of the reciprocating abrasion devices differ from each other in phase.

From a further aspect, the present invention also provides a method for evaluating abrasion resistance of a sample surface that includes causing abrasion of the sample surface with the sliding member of the above-described reciprocating tester, and evaluating a change in at least one characteristic of the sample surface by measuring the at least one characteristic before and after causing the abrasion.

In the reciprocating abrasion tester of the present invention, the sliding members of the reciprocating abrasion devices can reciprocate along sample surfaces at the same sliding speed. An average of the sliding speed can be 200 mm/sec or more. The sliding members are arranged such that reciprocating phases of the sliding members differ from each other, that is, reciprocation cycles of the reciprocating abrasion devices differ from each other. The reciprocating abrasion devices should include a mechanism to reciprocate the sliding members on the surfaces. Each of the devices may include a crank and a reciprocating arm.

When the tester includes two reciprocating abrasion devices, reciprocation cycles of the reciprocating abrasion devices preferably differ from each other in phase by 90° ± 30°.

On the other hand, when the number of the reciprocating abrasion devices is n (n denotes an integer of not less than 3), reciprocation cycles of the reciprocating abrasion devices preferably differ in phase from each other by 360°/n ± 30°.

It is preferable that the tester further includes a flywheel connected to the motor.

The tester may include a water supply mechanism. The water supply mechanism is preferably capable of supplying muddy water.

In the evaluating method of the present invention, the sliding member preferably reciprocates on the sample surface at an average sliding speed of 200 mm/sec or more. The abrasion may be caused while supplying muddy water onto the sample surface. The sample surface can be a surface of a glass sheet that is coated with a water repellent film.

In the tester of the present invention, changes in the load applied to the motor can be reduced. Accordingly, changes in the rotation of the motor can be reduced, thus allowing the occurrence of vibrations to be reduced.

Preferably, the flywheel is attached to a rotary shaft of the motor either directly or indirectly. The flywheel increases the inertial force of the motor, thus allowing the changes in the rotation of the motor to be further reduced. It is to be noted here that, although vibrations can be reduced when the flywheel is attached to an abrasion tester with only one reciprocating abrasion device, a sufficient effect cannot be obtained.

Further, by supplying water to the sample surface from the water supply mechanism, a state during rainfall can be imitated. By supplying muddy water to the sample surface from the water supply mechanism, a state where muddy water from a puddle adheres to the sample surface can be imitated.

Furthermore, by providing a plurality of the reciprocating abrasion devices in one reciprocating abrasion tester, it becomes possible to evaluate a plurality of samples at the same time.

Preferred embodiments of the invention will now be described, by way of example only, and with reference to the accompanying drawings in which:
FIG. 1 is a top view of an abrasion tester according to one example of the present invention.
FIG. 2A is a side view of an abrasion tester according to one example of the present invention, and FIG. 2B is a side view of the same in an elevated condition.
FIG. 3 is an enlarged view of a reciprocating abrasion device.
FIG. 4 is a top view of an abrasion tester according to another example of the present invention.
FIG. 5A is a side view of an abrasion tester according to another example of the present invention, and FIG. 5B is a side view of the same in an elevated condition.
FIG. 6A is a top view of an abrasion tester according to a preliminary consideration, and FIG. 6B is a side view of the same in an elevated condition.
FIG. 7 is a view illustrating a water supply mechanism.
FIG. 8 is a view illustrating measuring points for contact angles in Example.

### (Example 1)

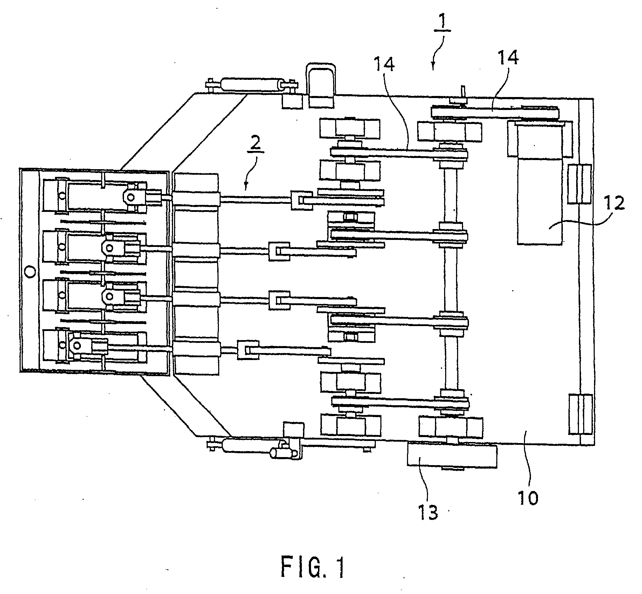

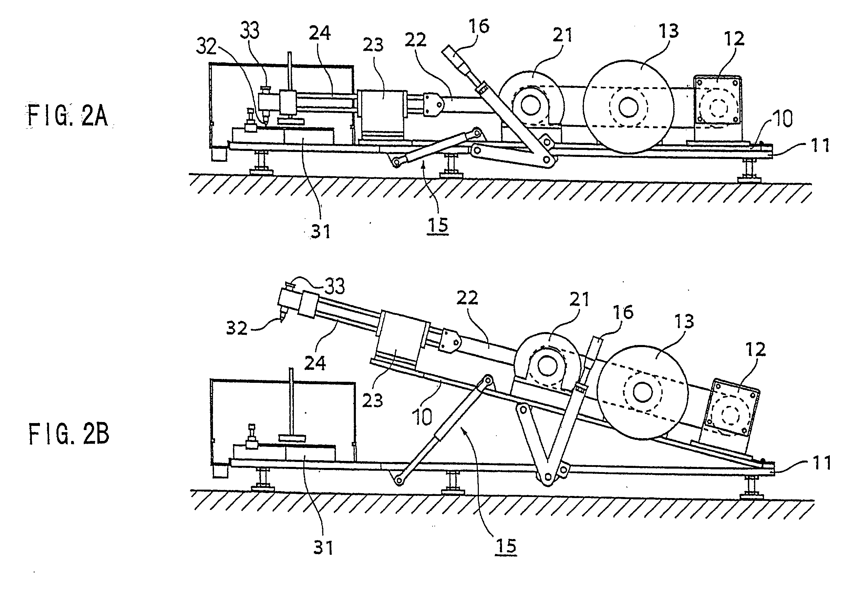

FIG. 1 and FIG. 2A show a top view and a side view of one embodiment of an abrasion tester according to the present invention, respectively. This abrasion tester includes a plurality of reciprocating abrasion devices connected to one motor. In the present Example, four reciprocating abrasion devices are provided. Further, a flywheel is attached to the motor indirectly.

This abrasion tester 1 includes a motor 12 and reciprocating abrasion devices 2 on a top base 10, and a sample stage 31 on a bottom base 11. A driving force from the motor 12 is transmitted to a rotary shaft to which a flywheel 13 is attached via a belt 14, and further transmitted to the reciprocating abrasion devices 2 via other belts 14. Then, the driving force is converted into a reciprocating motion by a crank 21 and a connecting rod 22. A shaft 24 is supported by a slide bearing 23. A sliding member 32 is mounted at the end of the shaft 24 and reciprocates.

Preferably, the motor 12 is provided with the flywheel 13 as in the present example. Rotation of the motor becomes smoother since the flywheel 13 increases the inertial force of the motor 12. The flywheel may be attached to the rotary shaft of the motor directly.

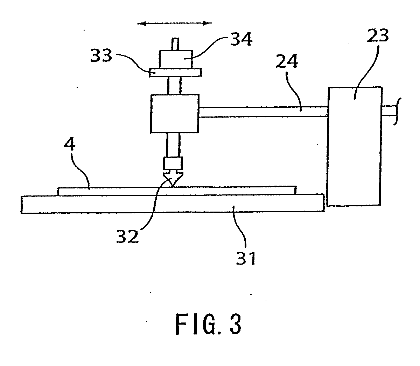

As shown in FIG. 3, in the present example, a wiper blade is used as the sliding member 32. Further, a weight 34 is set on a loading member 33 to adjust a load on a sample 4.

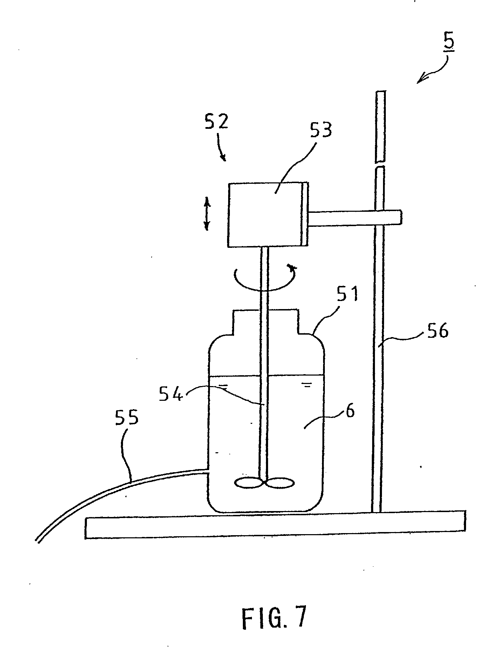

To the sample 4 disposed on a sample stage 31, water can be dripped from a water supply mechanism 5 shown in FIG. 7 through a tube 55. When the water supply mechanism 5 employs a tube pump as a pump, muddy water easily can be supplied to the sample.

The water supply mechanism 5 includes a stand 56 and a stirring mechanism 52 mounted thereto. The stirring mechanism 52 includes a motor 53 and a screw 54. A tank 51 contains muddy water 6, which is stirred by the stirring mechanism 52. The muddy water 6 is supplied to a pump, which is not shown in the drawing, via the tube 55. When the abrasion tester 1 includes the water supply mechanism 5 as described above, an abrasion test can be conducted under the conditions even more similar to those during actual use.

The abrasion tester 1 preferably includes a mechanism 15 for elevating the reciprocating abrasion devices 2 (hereinafter, referred to as an "elevating mechanism 15") for easy replacement of samples. FIG. 2B is a view illustrating an operation of the elevating mechanism 15. The top base including the motor 12 and the reciprocating abrasion devices thereon is elevated by pulling a lever 16, thus allowing the sample 4 on the sample stage 31 to be replaced with another one easily.

The reciprocation cycles of the plurality of reciprocating abrasion devices 2 differ from each other in phase. For example, when the abrasion tester includes two reciprocating abrasion devices, the phase difference may be set to 90° so as to prevent two wiper blades from turning around at the same time. As a result, changes in the load applied to the motor are reduced, thereby reducing the occurrence of vibrations. The same effect can be obtained when the phase difference is in the range of 90° ± 30°.

When the abrasion tester includes three reciprocating abrasion devices, the phase difference is set to 120°. On the other hand, when the abrasion tester includes four reciprocating abrasion devices, the phase difference may be set to 90°. When the number of the reciprocating abrasion devices is four or more, turning around of the devices at the same time does not have a significant effect. In this case, the devices should be arranged such that phase differences between the devices are about the same. That is, when the number of the reciprocating abrasion devices included in the abrasion tester is n (n denotes an integer of not less than 3), the phase difference should be set to 360°/n. The same effect can be obtained when the phase difference is in the range of 360°/n ± 30°.

The changes in the load applied to the motor can be reduced further by increasing the number of the reciprocating abrasion devices, as long as they can be driven by one motor.

Further, the more reciprocating abrasion devices in the abrasion tester, the more samples can be evaluated at the same time.

Examples of specifications for an abrasion tester according to the present example are shown in Table 1 below.

**Table 1**

| | |
|---|---|
| Stroke | 50 to 120 mm |
| Applicable blade width | 50 mm or less |
| Load range | 60 to 400g |
| Reciprocating speed | 3 cycles/second (equivalent to an average speed of 720 mm/s or less) |
| Supply of muddy water | 30 ml/min or less |

### (Example 2)

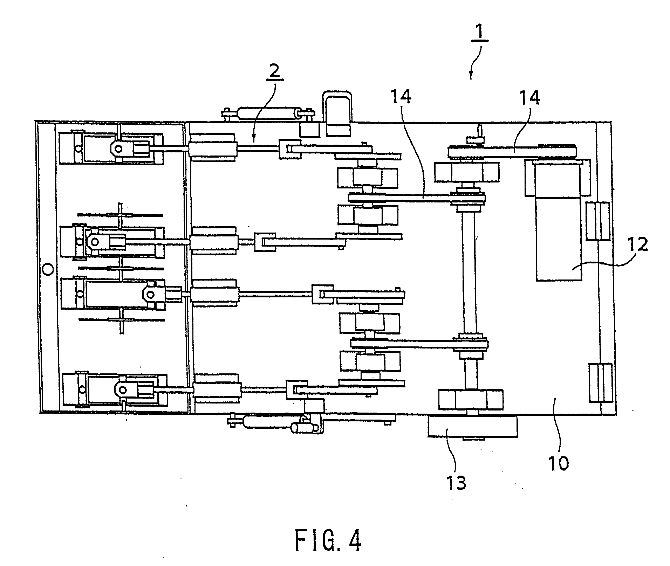

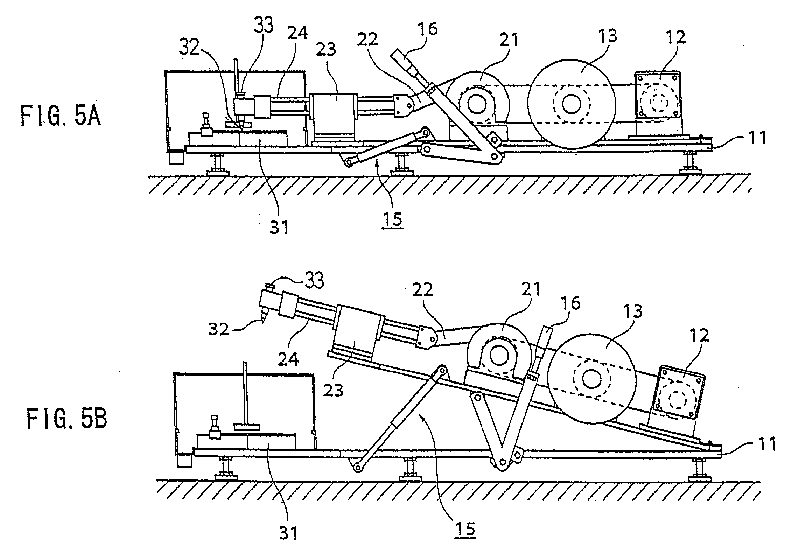

FIG. 4 and FIG. 5 show a top view and a side view of an abrasion tester in which a driving force is transmitted in a different manner from that in the abrasion test according to Example 1, respectively. Thus, the driving force from the motor 12 is transmitted to a rotary shaft to which a flywheel 13 is attached via a belt 14. The driving force is then further transmitted by further belts 14 to the reciprocating abrasion devices 2 in such a way that each further belt 14 supplies two reciprocating abrasion devices 2. This is in contrast to the embodiment of Fig. 1 in which each reciprocating abrasion device 2 is supplied by a separate belt 14. The specifications of the abrasion tester according to the present example are the same as those of the abrasion tester according to Example 1.

### (Comparative Example)

As a Comparative Example, measurements were taken using the abrasion tester fabricated according to the above-mentioned preliminary consideration. The specifications of this abrasion tester are the same as those of the abrasion tester according to Example 1 except that only one reciprocating abrasion device is provided therein, and corresponding numerals are used in FIGs. 6A to 6B.

### (Measurements and Results)

A sample is prepared in the following manner. First, a glass sheet of 150 × 50 × 3.4 mm was provided. A water repellent treatment solution as described later was coated onto the glass sheet by flow coating and then dried at room temperature for about 1 minute. A water repellent film thus was formed on the glass sheet. An abrasion test was conducted with respect to the thus-obtained sample under the following conditions: the stroke was 120 mm; a load of 100 g was applied per blade length of 500 mm; the reciprocating speed was 2.5-time reciprocation/sec; and 10 ml/min of muddy water prepared in the following manner was supplied to the sample.

The muddy water was prepared by mixing 2.5 g of test powder (I), type 2 in JIS (Japanese Industrial Standard), 1.0 g of test powder (I), type 7 in JIS, and 1000 ml of water and stirring the resultant mixture. The muddy water thus obtained was dripped on the surface of the sample by a muddy water supply mechanism as shown in FIG. 7. The muddy water in the tank 51 was stirred constantly by the stirring device 52 to prevent precipitation so that muddy water of the same quality could be supplied at all times (see FIG. 7).

As the pump for providing muddy water, a tube pump, which is not shown in the drawing, was used. The tube pump is suitable for use in the muddy water supply mechanism since it is less prone to be clogged with muddy water because of its structure. The muddy water 6 was dripped on the surface of the sample from the pump through a tube 55.

The water repellent treatment solution was prepared in the following manner. First, 100 g of ethanol was mixed with 0.02 g of heptadecafluorodecyltrimethoxysilane (CF₃(CF₂)₇(CH₂)₂Si(OCH₃)₃) and 0.3 g of tetraethoxysilane (Si(OCH₂CH₃)₄). The resultant mixture was stirred for 30 minutes, and then, 2.0 g of concentrated hydrochloric acid was added with agitation. The water repellent treatment solution was thus obtained.

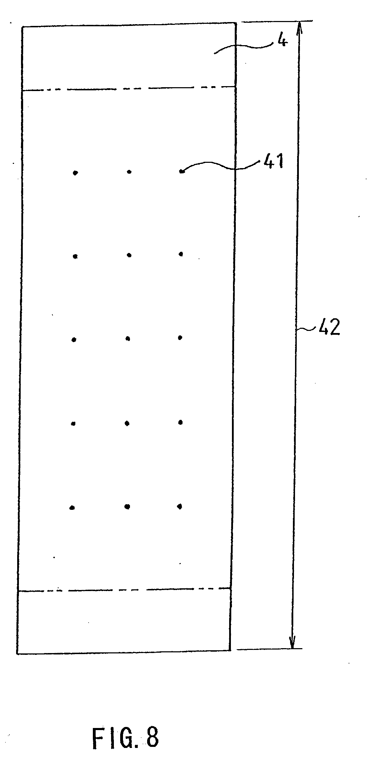

Then, static contact angles were measured at the respective measuring points 41 shown in FIG. 8. In FIG. 8, reference numeral 42 denotes a region where the sliding member slides. The results of the measurement are shown in Table 2 below. Before conducting the abrasion test, the average contact angle was 110°.

**Table 2**

| | Contact angle (°) | | | |
|---|---|---|---|---|
| | Maximum | Minimum | Average | Standard Deviation |
| Specific Example | 99.1 | 84.8 | 93.76 | 3.27 |
| Comparative Example | 90.6 | 60.9 | 81.27 | 7.79 |

As apparent from these results, in the abrasion test using the abrasion tester according to one example of the present invention, the standard deviation is less than one-half the standard deviation in the abrasion test using the abrasion tester according to Comparative Example. Therefore, it can be said that the abrasion tester according to the present invention can conduct an abrasion test exhibiting smaller variations in measurement.

According to the present invention, the bounding action of the wiper blade can be reduced, and variations in the test results can be made smaller, thus allowing an abrasion test with improved reliability to be conducted.

In addition, the abrasion tester according to the present invention enables a plurality of samples to be tested at the same time.

## Claims

1. A reciprocating abrasion tester (1) comprising:
a motor (12);
reciprocating abrasion devices (2) connected to the motor, each of the reciprocating abrasion devices comprising a sliding member (32) for causing abrasion of a sample surface; and
a loading member (33) for applying a load to the sliding member,
wherein reciprocation cycles of the reciprocating abrasion devices differ from each other in phase.

2. The reciprocating abrasion tester (1) according to claim 1, wherein each of the reciprocating abrasion devices (2) further comprises a crank (21) and a reciprocating arm (22).

3. The reciprocating abrasion tester (1) according to claim 1 or claim 2, wherein the reciprocating abrasion tester comprises two reciprocating abrasion devices (2), and reciprocation cycles of the reciprocating abrasion devices differ from each other in phase by 90° ± 30°.

4. The reciprocating abrasion tester (1) according to claim 1 or claim 2, wherein the reciprocating abrasion tester comprises a number n of the reciprocating abrasion devices (2), and reciprocation cycles of the reciprocating abrasion devices differ from each other in phase by 360°/n ± 30°, where n denotes an integer of not less than 3.

5. The reciprocating abrasion tester (1) as claimed in any one of claims 1 to 4, further comprising a flywheel (13) connected to the motor (12).

6. The reciprocating abrasion tester (1) as claimed in any one of claims 1 to 5, further comprising a water supply mechanism (5).

7. The reciprocating abrasion tester (1) as claimed in any one of claims 1 to 6, wherein the sliding member (32) includes a wiper blade.

8. A method for evaluating abrasion resistance of a sample (4) surface comprising causing abrasion of the sample surface with the sliding member (32) of the reciprocating abrasion tester (1) as claimed in any one of claims 1 to 7, and
evaluating a change in at least one characteristic of the sample surface by measuring the at least one characteristic before and after causing the abrasion.

9. The method according to claim 8, wherein the sliding member (32) reciprocates on the sample (4) surface at an average sliding speed of 200 mm/sec or more.

10. The method according to claim 8 or claim 9, wherein the abrasion is caused while supplying muddy water (6) onto the sample (4) surface.

11. The method as claimed in any one of claims 8 to 10, wherein the sample (4) surface is a surface of a glass sheet that is coated with a water repellent film.
